(19) Europäisches Patentamt

European Patent Office

Office européen des brevets

(11) **EP 0 696 215 B1**

(12) **FASCICULE DE BREVET EUROPEEN**

(45) Date de publication et mention
de la délivrance du brevet:
**05.01.2000 Bulletin 2000/01**

(51) Int. Cl.$^7$: **A61N 1/36**

(21) Numéro de dépôt: **94913668.3**

(22) Date de dépôt: **20.04.1994**

(86) Numéro de dépôt international:
**PCT/FR94/00442**

(87) Numéro de publication internationale:
**WO 94/23791 (27.10.1994 Gazette 1994/24)**

(54) **DISPOSITIF ELECTRONIQUE DESTINE A LA STIMULATION ADRENERGIQUE DU SYSTEME SYMPATHIQUE RELATIF A LA MEDIA VEINEUSE**

ELEKTRONISCHE VORRICHTUNG ZUR ADRENERGISCHEN REIZUNG DES SYMPATHISCHEN NERVENSYSTEMS IM VENÖSEN UMFELD

ELECTRONIC DEVICE FOR ADRENERGICALLY STIMULATING THE SYMPATHETIC SYSTEM WITH RESPECT TO THE VENOUS MEDIA

(84) Etats contractants désignés:
**AT BE CH DE ES FR GB IE IT LI LU NL**

(30) Priorité: **21.04.1993 FR 9304687**

(43) Date de publication de la demande:
**14.02.1996 Bulletin 1996/07**

(73) Titulaire:
**Klotz, Antoine Olivier**
**F-78580 Maule (FR)**

(72) Inventeur:
**Klotz, Antoine Olivier**
**F-78580 Maule (FR)**

(74) Mandataire: **Keib, Gérard et al**
**NOVAMARK TECHNOLOGIES,**
**"Anciennement Brevets Rodhain & Porte",**
**IMMEUBLE VICTORIA MICHELET,**
**122, rue Edouard Vaillant**
**92593 Levallois Perret Cedex (FR)**

(56) Documents cités:
**EP-A- 0 057 561**       **EP-A- 0 148 312**
**EP-A- 0 316 280**       **EP-A- 0 425 673**
**WO-A-87/00760**        **WO-A-88/07392**
**FR-A- 2 541 119**       **US-A- 4 167 189**

**Description**

La présente invention concerne un dispositif électronique destiné à la stimulation adrénergique du système sympathique relatif à la média veineuse.

Les maladies relatives à la stase veinolymphatique et au système vasculaire sont extrêmement répandues. Les études épidémiologiques montrent par exemple que la maladie thrombo-embolique constitue la troisième cause de mortalité dans les pays industrialisés et que parmi les personnes de plus de 30 ans, une sur deux souffre de maladie prévariqueuse. En outre, tous les sportifs de bon et de haut niveau ont intérêt à utiliser des méthodes physiques pour améliorer le trophisme cellulaire, récupérer rapidement après un effort, éliminer oedèmes, hématomes ou tendinites.

Les préventions et les soins les plus fréquemment utilisés sont essentiellement médicamenteux, fréquemment doublés d'analyses et de contrôles destinés à vérifier leur action. L'expérimentation clinique a permis de montrer l'intérêt, mais aussi les limites de ces méthodes, dont l'utilisation est très coûteuse et implique des risques allergiques ou hémorragiques qui s'ajoutent à leur taux d'efficacité encore insuffisant.

C'est pourquoi on cherche à développer des méthodes physiques de traitement susceptibles de compléter ou de remplacer avec une efficacité supérieure, l'utilisation des médicaments.

Des résultats intéressants ont été obtenus par la compression pneumatique séquentielle au moyen de bottes comprimant et décomprimant les membres inférieurs à un rythme permettant d'obtenir une amélioration des flux. L'usage de ces bottes, a donné des effets positifs. Cependant, ces appareillages sont complexes à mettre en oeuvre, non stériles et leur efficacité est limitée par l'absence d'effet profond.

On a déjà décrit un certain nombre d'appareils destinés à stimuler le triceps sural "Polock", mais ceux-ci ne peuvent pas être utilisés lorsque le malade est réveillé.

On peut également citer l'appareil décrit dans le brevet allemand n° 976 354 (Gratzel) qui se rapporte à la stimulation des muscles striés au moyen d'impulsions électriques.

De même le brevet français n° 2 493 437 (Baulande) concerne un appareil et un procédé pour stimuler la contraction des muscles striés par l'utilisation d'impulsions électriques unidirectionnelles visant à activer la pompe physiologique du mollet selon les indications thérapeutiques proposées antérieurement par PROWS et KAKKAR.

Toutefois le brevet Baulande ne décrit aucun moyen permettant de contrôler l'efficacité du traitement au moment où celui-ci est administré ou d'assurer la sécurité du patient à l'égard des courants électriques parasites.

Dans le brevet européen n° 0137007, le présent déposant a décrit un dispositif de stimulation des muscles lisses du tissu vasculaire garantissant une bonne sécurité de fonctionnement aux moyens de filtres reliés aux parties en contact avec le corps du patient et à l'alimentation en courant du dispositif. Durant le traitement, la régulation en courant est effectuée en fonction d'informations provenant de capteurs placés sur le corps du patient tels que des jauges thermiques, rhéologiques, résistives, ou myographiques, des sondes Doppler, etc.

L'expérimentation clinique a démontré l'originalité et l'efficacité de cet appareil. Toutefois cet appareil ne permet pas de suivre de façon souple et fiable l'efficacité du traitement au moment même de l'application de celui-ci.

Les capteurs placés sur le corps du malade qui peuvent indiquer l'efficacité globale du traitement ne permettent pas néanmoins de suivre de façon précise et instantanée l'évolution exacte des besoins du malade au fur et à mesure du traitement.

A défaut d'un contrôle instantané des effets du traitement, celui-ci ne peut pas être adapté à chaque instant de façon exacte aux besoins du patient. Il s'ensuit que la durée du traitement est souvent insuffisante ou au contraire excessive et que les paramètres caractérisant les impulsions électriques (notamment la tension), traversant le corps du patient ne conviennent pas tout à fait à la nature et à l'amplitude des maux dont celui-ci souffre.

La présente invention vise donc à pallier ces inconvénients en proposant un dispositif électronique destiné à la stimulation adrénergique du système sympathique relative à la média veineuse, comportant des moyens pour générer des impulsions électriques entre au moins deux électrodes disposées de façon appropriée sur le corps du patient, l'intensité, la tension, la forme et la fréquence des impulsions électriques étant réglables.

Selon l'invention, le dispositif électronique comporte des moyens pour mesurer l'impédance entre les électrodes.

En effet la masse cytoplasmique intra et extra cytologique peut être assimilée à un gel hétérogène constitué d'eau et de protéines pour environ 85 à 90% de son poids. On a remarqué que les troubles et les maladies précités provoquaient localement une variation de l'impédance de la masse cytoplasmique notamment mesurable en fonction des paramètres de fréquence et d'énergie de la stimulation donnée. Ainsi la présence d'un hématome ou d'un oedème peut représenter une différence d'impédance de 250 à 800 Ω environ tandis qu'un thrombus en formation pourra donner une différence d'impédance de ± 100 à 400 Ω.

De même, alors que les membres inférieurs d'un sportif en bonne forme présentent chacun une impédance variant entre 800 et 1200 Ω, cette impédance s'élève à un niveau situé entre 1200 et 3000 Ω en cas de fatigue importante après un effort ou en présence de contractures dues à la présence de résidus de la combustion musculaire et de lactate provoquant un oedème qui inhibe les fonctions de retour lymphatiques et veineux.

La mesure de l'impédance locale de la masse cytoplasmique permet ainsi de caractériser l'importance, la nature et le lieu exact des troubles et de fournir au patient un traitement correspondant exactement à ses besoins et à ses capacités.

Plus précisément l'invention autorise un double suivi des résultats des soins. Durant une séance de traitement, le suivi de l'évolution instantanée de l'impédance rend compte de la distensibilité et de la compliance veineuse. Sur l'ensemble du traitement, la variation absolue de l'impédance indique le tonus veineux. Ce double suivi de l'impédance de la masse cytoplasmique permet d'optimiser à double titre la nature des soins au patient.

Ces impulsions provoquent la stimulation adrénergique du système sympathique relatif à la média veineuse, source principale de noroadrénaline. Par activation des récepteurs alpha-adrénergiques postsynaptiques on induit une vaso constriction. Les effets incluent également l'accélération du retour veino-lymphatique et l'émulation du tissu pariétal assurant le tonus de la tunique adventicielle qui contrôle la distensibilité, la compliance et le diamètre veineux.

Un des avantages de l'invention est que la mesure d'impédance peut s'appliquer directement à toute la masse cytoplasmique comprise entre les électrodes fournissant le traitement. Il peut s'agir de la masse totale des membres ou d'une portion du corps du patient quel que soit le volume de cette portion, et non d'une section seulement de masse cytoplasmique comme il est d'usage dans les mesures rhéographiques d'impédance bien connues en exploration médicale fonctionnelle. Il n'est pas nécessaire d'utiliser d'autres électrodes pour cette mesure, ce qui facilite l'emploi de l'appareil. De plus, cette mesure d'impédance est effectuée sans recherche d'impédance particulière.

Suivant une version avantageuse de l'invention, le dispositif comporte des moyens pour régler la tension des impulsions en fonction du résultat de la mesure de ladite impédance.

De cette façon, on simplifie pour l'opérateur la mise en oeuvre d'un traitement adapté aux besoins du malade.

Suivant une version préférée du dispositif, celui-ci comporte des moyens d'asservissement de la tension des impulsions en fonction du résultat de la mesure instantanée de ladite impédance.

De la sorte, l'adaptation du traitement aux besoins du patient s'effectue de façon automatique et optimale, libérant en plus l'opérateur d'une surveillance et d'une opération techniques répétitives. Au besoin, le traitement peut donc être fourni au malade par une personne ne disposant pas de qualifications techniques approfondies telle une aide soignante ou le malade lui-même.

Suivant une autre version préférée de l'invention, le dispositif comprend un lecteur permettant l'échange de données avec un support de données amovibles, tel qu'une carte à mémoire et les moyens d'asservissement de la tension des impulsions utilisent des données enregistrées sur ledit support amovible.

Chaque patient dispose par exemple d'un tel support amovible qui comprend notamment les données médicales nécessaires au traitement du malade. L'utilisation de ces données par le dispositif peut permettre le réglage automatique des paramètres du traitement à fournir en fonction des besoins du malade et des séances de traitement déjà effectuées. Le maniement du dispositif est donc davantage automatisé dans le sens d'une adaptation encore meilleure aux besoins du malade et d'un allègement des tâches de l'opérateur.

D'autres caractéristiques et avantages de l'invention apparaîtront encore dans la description qui va suivre d'une réalisation préférée de l'invention, donnée à titre d'exemple non limitatif.

Aux dessins annexés:

- la figure 1 est une vue en perspective présentant le dispositif selon l'invention;
- la figure 2 est un schéma présentant les principaux éléments électroniques du dispositif et le principe général de leur organisation,
- la figure 3 présente une forme d'impulsion générée par le dispositif.

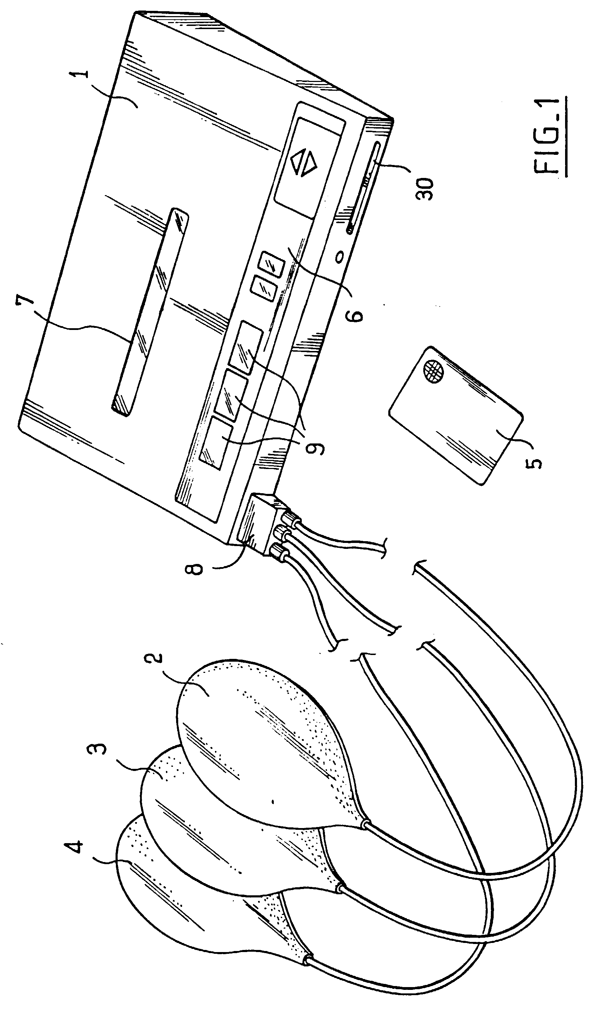

Le dispositif selon l'invention, présenté à la figure 1, se compose d'un appareil 1, de trois électrodes 2, 3 et 4, d'une carte à mémoire 5 détenue en propre par chaque malade et d'un capteur impédance métrique.

Le dispositif permet de faire circuler dans le corps du patient des séries d'impulsions électriques d'intensité en courant, d'amplitude en tension, de forme et de fréquence choisies, qui seront décrites plus loin.

L'appareil 1 a une forme rectangulaire plate. Il comporte un clavier de commande muni des touches de commandes visuelles en liaison avec les différentes fonctions de l'appareil (entrée et sortie de la carte à mémoire, mise en marche,...). Il comprend également trois touches 9 formant clavier digitosensitif. Celui-ci peut être utilisé comme un potentiomètre linéaire, un effleurement du doigt sur l'alignement constitué par les trois touches permettant de régler l'amplitude des impulsions à la valeur voulue. L'appareil 1 est aussi muni d'un écran d'affichage 7 à cristaux liquides permettant d'afficher sur deux lignes des instructions de commande ou des résultats de mesure par exemple. La fiche de connexion 8 relie les câbles des trois électrodes 2, 3, 4 à l'appareil 1.

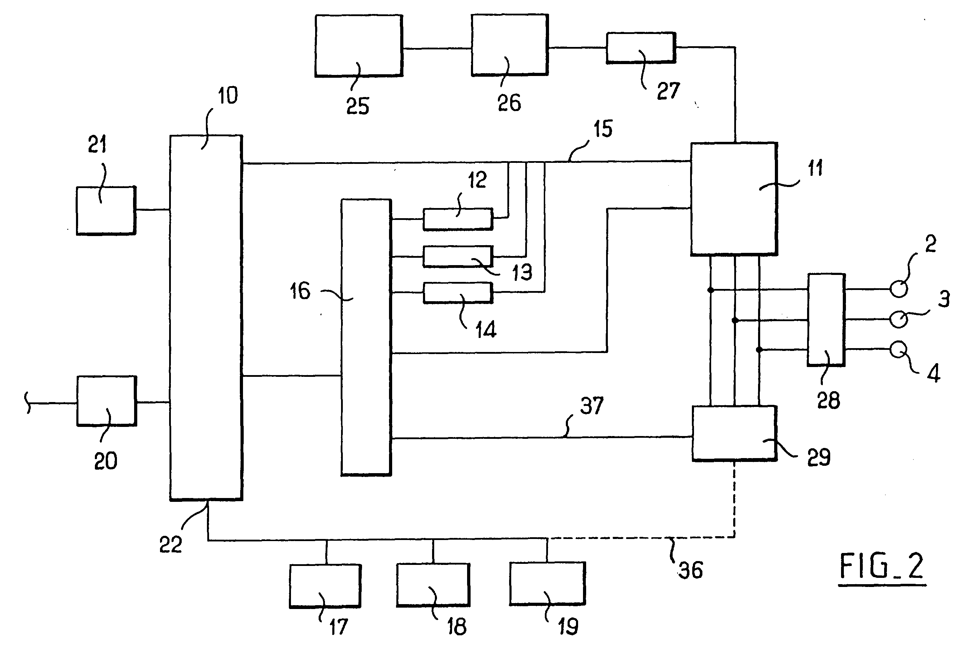

Les éléments électroniques de l'appareil 1 nécessaires à la génération et à la circulation des impulsions sont courants et largement connus dans l'état de la technique. Un grand nombre de ces éléments sont déjà décrits dans le document EP 0 137 007. On rappellera seulement ici les principaux d'entre eux, à l'aide de la figure 2. L'organisation générale de ces éléments électroniques est également à la portée de tout électronicien et s'inspire largement de celle décrite dans le document sus-mentionné. Seul le principe général de cette organisation est illustré en figure 2.

L'appareil comprend tout d'abord un microprocesseur 10 contrôlant la génération et la circulation des impulsions en fonction des données reçues. Le microprocesseur 10 comprend notamment de façon intégrée un générateur de signal carré, ce signal comprenant les données nécessaires à la génération des impulsions. Ce signal carré est envoyé dans le convertisseur numérique-analogique 11 qui délivre les impulsions circulant dans le corps du patient.

Le microprocesseur est classiquement assisté d'une horloge 12, d'une mémoire vive (RAM) et d'une mémoire morte (ROM), toutes deux symbolisées par le module 13, et d'un module d'interface 14 avec l'écran d'affichage 7. L'horloge 12, les mémoires 13 et module 14 sont reliés, d'une part, au bus 15 joignant le microprocesseur 10 au convertisseur numérique-analogique 11 et d'autre part, par l'intermédiaire d'un décodeur 16, au microprocesseur 10. Le microprocesseur est également relié au module d'interface 17 du clavier 6, au module d'interface 18 du clavier digitosensitif 9 et à la fiche de connexion 19 du capteur impédance métrique.

Le microprocesseur 10 est encore relié à un connecteur 20 permettant de raccorder l'appareil aux périphériques usuels couramment utilisés en informatique tels qu'une imprimante, un Modem, un ordinateur ou un minitel, notamment par un émetteur infrarouge ou ondes courtes.

Enfin le microprocesseur 10 est relié à un lecteur 21 de support de données amovible 5. La fente d'introduction 30 du support de données dans le lecteur est montrée en figure 1. Celui-ci peut être une disquette informatique ou préférentiellement une carte à mémoire classique telle qu'une carte à puce 5 ou une carte magnétique. Par exemple, il peut s'agir d'une carte à mémoire, carte à microcontrôleur, carte PCMCIA, etc... Cette carte 5 peut être détenue en propre par le patient dont elle contient notamment le dossier médical et épidémiologique. Y figurent toutes les données relatives au déroulement du traitement du patient (durée, nombre de séances effectuées, paramètres des impulsions, particularités du patient, valeur d'impédance initiale des membres du patient,...). Ce support de données amovible pourra au besoin être lu par le lecteur d'un ordinateur ou autre.

L'alimentation en puissance du convertisseur numérique analogique 11 comprend tout d'abord une source de puissance 25 de basse tension avantageusement constituée par une batterie ou un groupe de piles électriques. Cette source de puissance alimente également les autres composants de l'appareil, (ces alimentations n'ont pas été représentées), de sorte que l'ensemble de l'appareil est rendu autonome et portable.

La source de puissance 25 est reliée à un générateur de tension 26 comprenant un transformateur intégré non représenté, capable d'élever à 100 volts la basse tension provenant de la source 25. L'intensité maximale du courant délivré par ce transformateur est dans tous les cas inférieure à 3 milliampères. L'intensité des impulsions n'est donc jamais dangereuse pour le patient.

Le générateur de tension 26 est relié à un filtre 27 lui-même relié au convertisseur 11. Le rôle du filtre 27 est d'interdire le passage à travers le convertisseur 11 de courants accidentels dont l'intensité ou la tension viendraient parasiter le traitement ou représenteraient un danger pour le patient. Ce filtre 27 peut comprendre notamment un filtre passe-bande approprié.

Le convertisseur 11 est relié aux trois électrodes 2, 3 et 4 par l'intermédiaire d'un autre filtre 28 analogue. Le rôle de celui-ci est d'empêcher l'entrée par les électrodes 2, 3 et 4 de tout courant extérieur parasite susceptible de perturber le fonctionnement du dispositif ou le traitement du patient. Ces courants peuvent par exemple être ceux générés par un appareil de radiologie, un bistouri électrique, etc.

Le filtre 28 comprend notamment un relais fonctionnant de telle sorte qu'il reste ouvert tant que l'appareil n'a pas été convenablement programmé pour assurer le traitement du patient en toute sécurité.

Les électrodes 2, 3 et 4 sont constituées préférentiellement de feuilles de résine de silicone chargées de carbone conducteur. Le graphite qu'elles contiennent est disposé suivant une structure en couche ce qui facilite la circulation des électrons. Les électrodes ont une impédance très basse et sont très conductrices. Le câble qui permet de relier chaque électrode à l'appareil 1 a son extrémité noyée dans la masse de l'électrode. Les matériaux constituant câble et électrode les rendent autoclavables.

Une des caractéristiques de la présente invention est de comporter des moyens de mesure de l'impédance aux bornes des électrodes. Ces moyens sont ici constitués par un composant électronique 29, tel que le composant AD734AQ fabriqué par ANALOG DEVICE. De tels moyens ne sont pas nouveaux en eux-mêmes et sont largement connus en électronique. En revanche, leur emploi dans un dispositif tel que celui de l'invention est nouveau et produit des avantages considérables sur lesquels nous reviendrons.

Les trois électrodes sont disposées en différents endroits du corps du patient, l'électrode 3 jouant le rôle d'une électrode de référence. Le patient étant allongé sur le dos, on dispose par exemple l'électrode 2 sous son mollet gauche, l'électrode 4 sous son mollet droit et l'électrode 3 de référence sous son sacrum ou sur son pubis. Le contact entre la peau du patient et les électrodes est assuré par un gel approprié comme il est bien connu.

Les trois électrodes 2, 3 et 4 sont reliées via le filtre 28 au composant de mesure d'impédance 29. Celui-ci mesure l'impédance entre deux électrodes choisies selon le cas, reflet de la variation de la masse cytoplasmique correspondante.

Par exemple, l'impédance peut être mesurée entre les électrodes 2 et 4. Toutefois, il sera avantageux de mesurer l'impédance entre les électrodes 2 et 3 d'une part et 3 et 4 d'autre part. En effet par cette mesure différentielle de l'impédance, on obtient une mesure de l'impédance correspondant à chaque membre. Pour mesurer l'impédance entre deux électrodes, les données d'entrée fournies au composant 29 sont le potentiel de chaque électrode et le courant qui la traverse.

Le résultat de la mesure d'impédance est ensuite fourni en retour au microprocesseur 10. Deux voies d'acheminement sont envisageables pour cela: une première possibilité consiste à amplifier le résultat sous forme analogique et à le délivrer directement au port analogique 22 du microprocesseur 10. La connexion 36 correspondant à cette possibilité est figurée en trait pointillé sur la figure 2. Un tel procédé conduit à une erreur relative maximale variant de 2 à 4% entre le résultat calculé et le résultat transmis. La seconde possibilité consiste à convertir le résultat analogique en un résultat numérique, en préalable à son amplification. Une fois amplifié, ce résultat est transmis au microprocesseur 10 via le décodeur 16. Cette possibilité, représentée en trait plein sur la figure 2 par la connexion 37, produit une erreur relative située entre 0,1% et 1% en fonction de la qualité du convertisseur utilisé, ce qui est nettement suffisant.

La précision de la mesure est permise par une double calibration de l'appareil : les appareils sont une première fois calibrés de façon identique lors de la fabrication en usine. Ensuite au fur et à mesure de l'utilisation, par exemple à chaque utilisation, l'appareil effectue lui-même son propre calibrage, le microprocesseur 10 étant programmé pour opérer régulièrement un étalonnage interne de l'appareil au moyen d'une référence interne connue.

Cette double calibration entraîne que les mesures fournies pour un même patient par plusieurs dispositifs identiques selon l'invention sont identiques durant une période pouvant aller jusqu'à 2 ans après la sortie d'usine du dispositif. L'utilisation du même dispositif pour l'administration et le suivi du traitement d'un patient donné n'est donc pas nécessaire.

Une fois le résultat de la mesure d'impédance acquis par le microprocesseur 10, celui-ci adapte le signal carré destiné au convertisseur 11, de façon que l'amplitude en tension des impulsions fournies au patient tienne compte de la décroissance de l'impédance mesurée. Ces mesures et cette adaptation peuvent être effectuées à chaque instant de sorte qu'on réalise un asservissement de la tension des impulsions délivrées. On peut par exemple utiliser la formule

$$E = \frac{U^2}{R} \times t$$

pour maintenir l'énergie constante, U étant la tension, R l'impédance et t la durée des impulsions.

Les impulsions sont délivrées entre les électrodes 2 et 4 sous la forme de trains d'impulsions, uniques et répétitives à très basse fréquence, identiques toutes positives ou toutes négatives ou encore sous la forme de trains d'impulsions très avantageusement négatives et positives alternées. Elles peuvent aussi être délivrées sous la forme d'une série ininterrompue d'impulsions.

Au cours d'une séance de traitement, les impulsions générées entre les électrodes ont une intensité efficace constante et inférieure à 350 micro-ampères, avantageusement inférieure à 250 micro-ampères avec une énergie inférieure à 10 millijoules, un tel courant ne pouvant pas avoir d'action polarisante, ni électrisante, ni ionisante, ni agir sur une quelconque prothèse métallique, dispositif intra-utérin, cuprocontraceptif ou stimulateur cardiaque.

Le fait de travailler en courant constant et de faire varier l'amplitude des impulsions suivant l'évolution de l'impédance permet d'assurer des soins égaux durant tout le traitement quelles que soient les variations de la masse cytoplasmique durant le traitement.

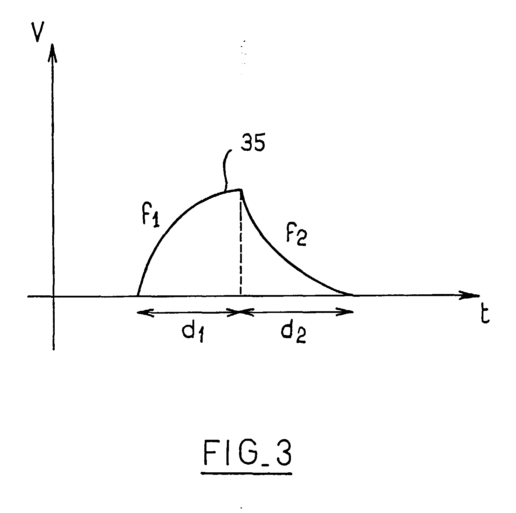

Un exemple d'impulsions 35 est illustré à la figure 3. L'axe des abscisses et l'axe des ordonnés représentent respectivement le temps et l'amplitude en tension V. La durée d'établissement d1 maximale des impulsions est de deux millisecondes. Leur durée d'extinction d2 est égale à la durée d'établissement d1 et la forme d'extinction F2 est l'inverse de la forme d'établissement F1. La courbe de montée est une courbe exponentielle avec une pente à l'origine de 200 volts par milliseconde. La fréquence des impulsions est inférieure à 2 Hz et de préférence voisine de 1,75 Hz.

L'amplitude en tension des impulsions varie entre 0 et 150 V ou encore -150 et 0 V et est soumise à l'asservissement décrit plus haut. Bien entendu, au besoin, l'amplitude des impulsions peut aussi être choisie manuellement à l'aide par exemple du clavier 6 et du clavier digitosensitif 9.

De façon avantageuse, l'intensité et la tension des impulsions peuvent être choisies de sorte que l'énergie d'une impulsion se situe entre 0 et 8 mJ. La polarité des impulsions peut être positive, négative ou alternativement positive et négative et ce suivant des combinaisons variées. La durée de la séance peut aller de 1 seconde à plusieurs jours si nécessaire, typiquement de 20 à 30 minutes ou le temps d'une intervention chirurgicale. Une gamme de fréquence avantageuse se situe entre 0,75 Hz et 2,75 Hz.

L'amplitude des impulsions peut être asservie en fonction de l'impédance mesurée, mais aussi grâce à l'utilisation de la carte à mémoire 5 en fonction des particularités du patient soigné. Cette carte échange diverses données techniques et médicales relatives au traitement et au patient avec l'appareil 1 par le biais du lecteur 21 et enregistre notamment au fur et à mesure tous les paramètres de l'ensemble du traitement. Elle permet une programmation automatique du dispositif au fur et à mesure de l'évolution du traitement et des besoins du malade. Il en résulte une grande facilité et une grande sécurité d'emploi du dispositif. Les données figurant sur cette carte peuvent également être utilisées dans le cadre d'études économiques, statistiques et épidémiologiques ainsi que pour la maintenance ou la télémaintenance de l'instrument. Eventuellement, le dispositif peut être conçu de sorte que la simple introduction de la carte à mémoire dans l'appareil 1 provoque la reprogrammation automatique de celui-ci de façon à générer des impulsions adaptées au patient.

Avantageusement, la carte à puce comporte en premier lieu :

- une zone système n° de carte, mois années, identification de service, n° de série etc...
- une zone secrète de cryptage des données interfacée avec le microcontrôleur de l'appareil qui assure la sécurité des soins et empêche à un non autorisé d'y accéder ;
- une zone d'interface avec les PC du commerce : Power PC, Power MAC, MAC OS, OS2, DOS, UNIX, etc...
- des niveaux d'autorisation : lecture seule, transmission, écriture instrument seul, écriture protocole d'ordonnance par un PC externe, nombres d'utilisations autorisées.

Elle comporte en deuxième lieu une zone d'identification : centre des soins, identité patient, type de maladie, de soins, nombre de soins, durée, horodatage, polarité, énergie, sexe, âge, avec en outre une quinzaine de paramètres utilisables dans le type de malade traité et notamment : les mesures de l'impédance du patient au début, après 5, 10, 15, 20, 30 minutes à la fin du traitement, impédance totale, droite, gauche.

Un tableau des mesures de la séance, et des séances antérieures, permet l'analyse par le praticien de l'état instantané du patient, de ses progrès par rapport à l'état antérieur, de ses facteurs de risque, notamment de thrombose.

Cette analyse de toutes les données significatives est visible sur l'écran de l'appareil pour le personnel soignant, peut être interprétée directement par l'appareil, lue par l'ordinateur externe du médecin ou de l'hôpital pour être collationnée dans une base de données permettant, sans autre saisie par le personnel soignant, une interprétation épidémiologique et statistique.

On y fera notamment le diagnostic de l'état statique ou dynamique de la masse acqueuse, protéique, graisseuse, de la masse de cellules vivantes, morbides ou mortes, de l'élimination et de sa vitesse, acqueuse ou protéique de l'oedème, de l'hématome ou du thrombus, de l'amélioration de la compliance veineuse.

En cas d'apparition d'un seuil de facteur de risque : thrombus ou oedème, l'instrument alerte immédiatement le soignant ou le soigné par un interface visuel et sonore, les actions appropriées sont alors déclenchées sans retard pour le patient.

La carte à puce est écrite par l'instrument lors de sa première mise en service sur le patient et saisit ensuite tous les événements et mesures. Elle devient alors l'interface de communication parfait pour un archivage durable (10 ans) et recyclable (ABS), pour l'appareil qu'elle programme alors automatiquement, sans autre intervention du personnel soignant, pour le PC du médecin qu'elle nourrit de ses informations afin de préparer son diagnostic comme elle lui permet la surveillance et l'assure de la réalité des soins (horodatage), pour l'interprétation épidémiologique acquise de façon factuelle précise et économique, pour la sécurité de l'information qu'elle protège en fonction des lois en vigueur.

La possibilité de connecter le dispositif, au moyen du connecteur 20, à une imprimante, un modem, un ordinateur ou un minitel trouve de nombreuses applications par exemple dans les cas de soins à distance ou pour le stockage et l'archivage des données.

Le dispositif selon l'invention peut également comporter un capteur de mouvement (non représenté) tel que celui commercialisé par INTERLINK. Le capteur a la forme d'un disque plat d'environ 20 millimètres de diamètre. Il est immobilisé sur le mollet du patient au moyen de tissu adhésif.

La circulation des impulsions électriques dans le corps du patient provoque une réaction physiologique de battement de la surface des membres soumis aux impulsions. Chaque tremblement produit une légère compression de la couche de carbone située à l'intérieur du capteur. Celle-ci est munie de deux électrodes internes reliées à l'appareil, permettant la circulation d'un courant. La compression fait varier la résistance de la couche de carbone dont la mesure est transmise au microprocesseur qui est ainsi informé de l'importance des battements.

Pour que le traitement ne soit ni excessif, ni insuffisant, les battements doivent être compris entre des seuils haut et bas connus par le microprocesseur 10 et qui peuvent être fonction du patient. Si l'un des seuils est franchi, le microprocesseur 10 modifie le signal carré délivré au convertisseur 11 pour modifier la tension des impulsions en vue de produire des battements compris entre les deux seuils haut et bas.

L'utilisation du capteur de mouvement permet de fournir un paramètre supplémentaire, l'importance des battements de la surface de la zone stimulée, pour asservir la tension des impulsions délivrées au patient et fournir à celui-ci le traitement le plus adapté à ses besoins.

Le dispositif permet de résorber ou de faire disparaître de nombreux troubles physiologiques.

Les avantages de la stimulation électrique de la média veineuse incluent, sans l'usage de médicaments:

-   la libération de molécules fibrinolytiques notamment du plasminogène et de l'ATP et une diffusion directe (sans passage par les synapses) sur les fibres musculaires striées déterminant par la répétition d'impulsions uniques à très basse fréquence, des compressions régulières sur les veines suivies d'une ample période de relâchement, à la manière d'une pompe péristaltique, induisant par leur battement physiologique le réflexe moteur des saccules du gant lymphatique;
-   une mémorisation progressive et durable par les veines et les muscles adjacents et la reprise d'un tonus optimum permettant la diminution du diamètre des veines et l'amélioration de leur compliance ;
-   l'accélération par un facteur 4 à 14 de la vitesse veineuse pour l'individu bénéficiant des soins de ce dispositif;
-   l'élimination totale de la stase veineuse reconnue comme la cause majeure de formation des thrombus;
-   la résorption des hémorroïdes et varices régressives de la femme parturiente;
-   l'élimination des douleurs, crampes et paresthésies dues au syndrome veino-lymphatique;
-   une accélération du retour veineux profond et superficiel, de la microcirculation de la vitesse lymphatique;
-   la repolarisation et la récupération de la forme et de la plasticité des érythrocytes et l'accroissement de la fluidité du sang;
-   une amélioration de la précharge cardiaque, la reprise de l'acte moteur déclenchant l'activité hormonale métabolique et vitale;
-   la reprise du trophisme et l'arrêt du catabolisme cellulaire des patients immobilisés;
-   l'élimination des oedèmes aqueux banals ou gravidiques, des oedèmes protéiques ou des hématomes, la lyse et la dispersion des agrégats de leucocytes, érythrocytes, thrombus, lactates et cristaux d'acide urique;
-   une accélération de la perfusion de l'oxygène et du glucose.

Ce dispositif est différent des stimulateurs neuro-musculaires ou neuro-physiologiques qui agissent sur les muscles striés du squelette par l'intermédiaire des synapses ou plaques motrices. Comme autres avantages dus à cette différence, le dispositif ne peut jamais avoir d'effet thermique, polarisant, ou tétanisant, ce qui permet de l'utiliser sur toutes catégories de patients sans contre-indications dues par exemple à la présence d'une prothèse métallique, d'un dispositif intra-utérin, cuprocontraceptif, ou de stimulateur cardiaque.

C'est un avantage de l'invention que de mesurer les variations entre des zones du corps provoquées par les stimulations subies pour donner une représentation précise ou déceler un facteur de risque de l'état du patient, notamment de la présence d'un obstacle à la circulation tel qu'un hématome, un oedème, un thrombus, par la mesure comparative ou différentielle de la masse des deux membres inférieurs. De même, une telle mesure appliquée à intervalles déterminés durant les soins donne une évaluation et une mesure qualitative du traitement reçu et de son efficacité.

Le rythme et la durée des séances dépend des besoins thérapeutiques et physiologiques du malade. Pour un sportif de haut niveau, après un effort important, trois séances de douze à quinze minutes chacune, au cours d'une journée, permettent de ramener l'impédance des membres du sportif à sa valeur initiale qui correspond à la remise en forme de l'athlète.

La genèse de courants haute tension par une alimentation basse tension de sécurité de capacité inférieure à 3 mA garantit la sécurité du patient.

Diverses modifications et améliorations du présent dispositif peuvent être effectuées, notamment au sujet du choix et de l'organisation des composants électroniques de l'appareil 1 ou des caractéristiques des impulsions circulant dans le corps du patient, sans sortir du cadre de l'invention.

## Revendications

1.   Dispositif électronique destiné à la stimulation adrénergique du système sympathique relatif à la média veineuse, comportant des moyens (10) pour générer des impulsions électriques entre au moins deux électrodes (2, 3, 4) disposables de façon appropriée sur le corps du patient, caractérisé en ce que ce dispositif comportant des moyens (2, 3, 4, 29) pour mesurer l'impédance entre les électrodes (2, 3, 4), et en ce que l'intensité, la tension, la forme et la fréquence des impulsions électriques (35) étant réglables, le dispositif comporte des moyens (10) pour régler la tension des impulsions (35) appliquées entre les électrodes (2, 3, 4) de façon à maintenir l'intensité desdites impulsions à une valeur constante durant la session de stimulation adrénergique.

2.   Dispositif conforme à la revendication 1, caractérisé en ce qu'il comporte des moyens pour régler la tension des impulsions (35) en fonction du résultat de la mesure de ladite impédance.

3.   Dispositif conforme à la revendication 1, caractérisé en ce qu'il comporte des moyens d'affichage (7, 19) du résultat de la mesure de ladite impédance.

**4.** Dispositif conforme à l'une des revendications 1 à 3, caractérisé en ce qu'il comporte des moyens d'asservissement (10, 36 ; 37) de la tension des impulsions (35) en fonction du résultat de la mesure instantanée de ladite impédance.

**5.** Dispositif conforme à l'une des revendications 1 à 4, caractérisé en ce que le dispositif comprend un lecteur (21, 30) permettant au dispositif d'échanger des données avec un support de données amovible (5) tel qu'une carte à mémoire.

**6.** Dispositif conforme aux revendications 4 et 5, caractérisé en ce que les moyens d'asservissement (10, 36 ; 37) de la tension des impulsions électriques (35) utilisent des données enregistrées sur ledit support amovible (5).

**7.** Dispositif conforme à l'une des revendications 1 à 6, caractérisé en ce qu'il comporte un capteur de mouvement analysant le battement de la surface d'une zone du patient soumise aux impulsions.

**8.** Dispositif conforme à l'une des revendications 4 ou 6, caractérisé en ce que les moyens d'asservissement de la tension des impulsions électriques utilisent les mesures fournies par un capteur de mouvement analysant le battement de la surface d'une zone du patient soumise aux impulsions.

**9.** Dispositif conforme à l'une des revendications 1 à 8, caractérisé en ce qu'il comporte des premiers moyens de filtrage (28) auxquels sont reliées des électrodes (2, 3, 4) interdisant l'entrée par les électrodes (2, 3, 4) de tout courant électrique extérieur susceptible de perturber le fonctionnement du dispositif ou le traitement du patient.

**10.** Dispositif conforme à l'une des revendications 1 à 9, caractérisé en ce qu'il comporte des seconds moyens de filtrage (27) reliés aux moyens d'alimentation en énergie (25, 26) du dispositif et interdisant la circulation entre les électrodes (2, 3, 4) d'un courant électrique différent du courant désiré.

**11.** Dispositif conforme à l'une des revendications 1 à 10, caractérisé en ce que les impulsions électriques (35) générées entre les électrodes (2, 3, 4) ont une intensité efficace inférieure à 350 micro-ampères.

**12.** Dispositif conforme à l'une des revendications 1 à 11, caractérisé en ce que la fréquence des impulsions électriques (35) générées entre les électrodes (2, 3, 4) est inférieure à 2 Hz.

**13.** Dispositif conforme à l'une des revendications 1 à 12, caractérisé en ce que le dispositif comporte trois électrodes (2, 3, 4), l'une (3) des trois électrodes constituant une électrode de référence qui permet une mesure comparée des impédances respectives entre celle-ci et chacune des deux autres électrodes (2, 4).

**14.** Dispositif conforme à l'une des revendications 1 à 13, caractérisé en ce que les électrodes (2, 3, 4) sont constitués par des feuilles en résine de silicone imprégnées de carbone.

**15.** Dispositif conforme à l'une des revendications 1 à 14, caractérisé en ce que les moyens d'alimentation en énergie (25, 26) du dispositif sont autonomes.

**16.** Dispositif conforme à l'une des revendications 1 à 15, caractérisé en ce que le dispositif comporte des moyens d'interface (20) permettant de relier le dispositif à des moyens informatiques tels qu'un ordinateur ou une imprimante.

**Claims**

**1.** ELectronic device intended for adrenergic stimulation of the sympathetic system with respect to the venous media, having means (10) for generating electrical pulses between at least two electrodes (2, 3, 4) which can be arranged suitably on the patient's body, characterized in that this device has means (2, 3, 4, 29) for measuring the impedance between the electrodes (2, 3, 4), and in that, the strength, the voltage, the shape and the frequency of the electrical pulses (35) being adjustable, the device has means (10) for adjusting the voltage of the pulses (35) which were applied between the electrodes (2, 3, 4) so as to keep the strength of the said pulses at a constant value during the adrenergic stimulation session.

**2.** Device according to Claim 1, characterized in that it has means for adjusting the voltage of the pulses (35) as a function of the result of the measurement of the said impedance.

**3.** Device according to claim 1, characterized in that it has means (7, 19) for displaying the result of the measurement of the said impedance.

**4.** Device according to one of Claims 1 to 3, characterized in that it has means (10, 36; 37) for slaving the voltage of the pulses (35) as a function of the result of the instantaneous measurement of the said impedance.

**5.** Device according to one of Claims 1 to 4, characterized in that the device comprises a reader (21, 30) allowing the device to exchange data with a removable data medium (5) such as a memory card.

6. Device according to claims 4 and 5, characterized in that the means (10, 36; 37) for slaving the voltage of the electrical pulses (35) use data recorded on the said removable medium (5).

7. Device according to one of Claims 1 to 6, characterized in that it has a motion sensor which analyses the pulsation of the surface of a region of the patient which is subjected to the pulses.

8. Device according to one of Claims 4 and 6, characterized in that the means for slaving the voltage of the electrical pulses use the measurements supplied by a motion sensor which analyses the pulsation of the surface of a region of the patient which is subjected to the pulses.

9. Device according to one of Claims 1 to 8, characterized in that it has first filtering means (28) to which electrodes (2, 3, 4) are connected which prevent input through the electrodes (2, 3, 4) of any external electric current liable to interfere with the operation of the device or the treatment of the patient.

10. Device according to one of Claims 1 to 9, characterized in that it has second filtering means (27) which are connected to the power supply means (25, 26) of the device and prevent an electric current other than the desired current from flowing between the electrodes (2, 3, 4).

11. Device according to one of Claims 1 to 10, characterized in that the electrical pulses (35) which are generated between the electrodes (2, 3, 4) have a root mean square strength of less than 350 microamperes.

12. Device according to one of Claims 1 to 11, characterized in that the frequency of the electrical pulses (35) which are generated between the electrodes (2, 3, 4) is less than 2 Hz.

13. Device according to one of Claims 1 to 12, characterized in that the device has three electrodes (2, 3, 4), one (3) of the three electrodes constituting a reference electrode which allows comparative measurement of the respective impedances between it and each of the other two electrodes (2, 4).

14. Device according to one of Claims 1 to 13, characterized in that the electrodes (2, 3, 4) consist of carbon-impregnated sheets of silicone resin.

15. Device according to one of Claims 1 to 14, characterized in that the power supply means (25, 26) of the device are autonomous.

16. Device according to one of Claims 1 to 15, characterized in that the device has interface means (20) for connecting the device to data-processing means such as a computer or a printer.

**Patentansprüche**

1. Elektronische Vorrichtung zur adrenergischen Reizung des sympathischen Nervensystems im venösen Umfeld umfassend eine Einrichtung (10) zum Erzeugen elektrischer Impulse zwischen mindestens zwei Elektroden (2, 3, 4), die zweckmäßig am Körper eines Patienten anbringbar sind, **dadurch gekennzeichnet, daß** diese Vorrichtung eine Einrichtung (2, 3, 4, 29) zum Messen der Impedanz zwischen den Elektroden (2, 3, 4) umfaßt und daß die Stärke der Spannung, die Form und die Frequenz der elektrischen Impulse (35) regelbar sind, wobei die Vorrichtung eine Einrichtung (10) zum Regeln der Spannung der Impulse (35) umfaßt, die zwischen den Elektroden (2, 3, 4) derart angelegt sind, daß die Stärke der Impulse auf einem konstanten Niveau während der Sitzung einer adrenergetischen Reizung aufrecht gehalten wird.

2. Vorrichtung nach Anspruch 1, **dadurch gekennzeichnet, daß** sie eine Einrichtung zum Regeln der Spannung der Impulse (35) in Abhängigkeit von dem Ergebnis der Impedanzmessung umfaßt.

3. Vorrichtung nach Anspruch 1, **dadurch gekennzeichnet, daß** sie eine Anzeigeeinrichtung (7, 19) für das Ergebnis der Impedanzmessung umfaßt.

4. Vorrichtung nach einem der Ansprüche 1 bis 3, **dadurch gekennzeichnet, daß** sie eine Regelungseinrichtung (10, 36; 37) der Spannung der Impulse (35) in Abhängigkeit von dem Ergebnis der Momentanmessung der Impedanz umfaßt.

5. Vorrichtung nach einem der Ansprüche 1 bis 4, **dadurch gekennzeichnet, daß** die Vorrichtung ein Lesegerät (21, 30) umfaßt, das es der Vorrichtung ermöglicht, Daten mit einem mobilen Datenträger (5), insbesondere einer Speicherkarte, auszutauschen.

6. Vorrichtung nach einem der Ansprüche 4 und 5, **dadurch gekennzeichnet, daß** die Regelungseinrichtung (10, 36; 37) der Spannung der elektrischen Impulse (35) die aufdem mobilen Träger (5) gespeicherten Daten verwendet.

7. Vorrichtung nach einem der Ansprüche 1 bis 6, **dadurch gekennzeichnet, daß** sie einen Bewegungssensor aufweist, der die Hin- und Herbewegung eines mit Impulsen behandelten Oberflächenbereichs des Patienten analysiert.

**8.** Vorrichtung nach einem der Ansprüche 4 oder 6, **dadurch gekennzeichnet, daß** die Regelungseinrichtung der Spannung der elektrischen Impulse die Messungen verwendet, die von einem die Hin- und Herbewegung des mit Impulsen behandelten Oberflächenbereichs des Patienten analysierenden Bewegungssensors erbracht sind.

**9.** Vorrichtung nach einem der Ansprüche 1 bis 8, **dadurch gekennzeichnet, daß** sie eine erste Filtereinrichtung (28) umfaßt, mit der die Elektroden (2, 3, 4) verbunden sind, um den Zugang von jedem äußeren elektrischen Strom, der zur Störung der Funktionsfähigkeit der Vorrichtung oder der Behandlung des Patienten fähig ist, über die Elektroden (2, 3, 4) zu verhindern.

**10.** Vorrichtung nach einem der Ansprüche 1 bis 9, **dadurch gekennzeichnet, daß** sie eine zweite Filtereinrichtung (27) umfaßt, die mit der Energieversorgungseinrichtung (25, 26) der Vorrichtung verbunden ist und die Zirkulation eines elektrischen Stromes, der sich gegenüber dem gewünschten Strom unterscheidet, zwischen den Elektroden (2, 3, 4) verhindert.

**11.** Vorrichtung nach einem der Ansprüche 1 bis 10, **dadurch gekennzeichnet, daß** die zwischen den Elektroden (2, 3, 4) erzeugten elektrischen Impulse (35) eine Wirkstärke von weniger als 350 Mikro-Ampere aufweisen.

**12.** Vorrichtung nach einem der Ansprüche 1 bis 11, **dadurch gekennzeichnet, daß** die Frequenz der zwischen den Elektroden (2, 3, 4) erzeugten elektrischen Impulse (35) kleiner als 2 Hz ist.

**13.** Vorrichtung nach einem der Ansprüche 1 bis 12, **dadurch gekennzeichnet, daß** die Vorrichtung drei Elektroden (2, 3, 4) umfaßt, wobei eine (3) der drei Elektroden eine Referenzelektrode bildet, die eine Vergleichsmessung der jeweiligen Impedanzen zwischen dieser und jeder der beiden anderen Elektroden (2, 4) ermöglicht.

**14.** Vorrichtung nach einem der Ansprüche 1 bis 13, **dadurch gekennzeichnet, daß** die Elektroden (2, 3, 4) durch Folien aus Silikonharz gebildet sind, die mit Kohlenstoff imprägniert sind.

**15.** Vorrichtung nach einem der Ansprüche 1 bis 14, **dadurch gekennzeichnet, daß** die Energieversorgungseinrichtung (25, 26) der Vorrichtung unabhängig ist.

**16.** Vorrichtung nach einem der Ansprüche 1 bis 15, **dadurch gekennzeichnet, daß** die Vorrichtung eine Schnittstelleneinrichtung (20) umfaßt, die eine Verbindung der Vorrichtung mit einer Datenverarbeitungseinrichtung, insbesondere einem Rechner oder einem Drucker, ermöglicht.

EP 0 696 215 B1

FIG_1

FIG_2

EP 0 696 215 B1

FIG_3